# EUROPEAN PATENT SPECIFICATION

(11) **EP 4 636 826 B1**
(45) Date of publication and mention of the grant of the patent: **18.11.2026**
(21) Application number: 24170844.5
(22) Date of filing: 17.04.2024
(51) Int. Cl.: A61B 6/10, A61L 2/08

(54) **RADIATION TOLERANT ELECTRONIC MEDICAL DEVICE**
STRAHLUNGSTOLERANTE ELEKTRONISCHE MEDIZINISCHE VORRICHTUNG
DISPOSITIF MÉDICAL ÉLECTRONIQUE TOLÉRANT AUX RAYONNEMENTS

(43) Date of publication of application: 22.10.2025
(73) Proprietor: TE Connectivity Solutions GmbH, 8200 Schaffhausen (CH)
(72) Inventor: POENARU, Ilie, 8200 Schaffhausen (CH); JAILLET, Romain, 8200 Schaffhausen (CH); BRUNNER, Ismael, 8200 Schaffhausen (CH); PERRIER, Christian, 8200 Schaffhausen (CH); DE SOUSA, Leticia, 8200 Schaffhausen (CH)
(74) Representative: Grünecker Patent- und Rechtsanwälte PartG mbB

(56) References cited:
- JP-A- 2014 122 375
- JP-B2- 6 125 827
- US-A1- 2003 169 582
- US-A1- 2004 056 334
- US-A1- 2009 202 387

## Description

Medical devices can be exposed to radiation for sterilization. For example, invasive medical devices can be irradiated with electromagnetic radiation or charged particle beams like electron beams before coming into contact with patients in order to reduce the risk of infection. Medical devices, including disposable medical devices are routinely sterilized before use. For example, an exterior surface of the device may couple to the environment, such as to tissue, blood, or a therapeutic fluid (e.g. intravenously administered fluid). Sterile coupling of the device and/or exterior surface to the environment reduces the risk of infection. Medical devices that include electronics may need to be sterilized. US 2009/202387 A1 discloses an implantable system having internal circuitry configured to withstand a pre-determined amount of sterilization radiation. JP 2014 122375 A proposes to provide a radiation shielding coating member with high durability that prevents corrosion and, at the same time, does not experience a decline in mechanical strength, corrosion resistance, or radiation shielding performance due to exposure to gamma rays or X-rays or a humid environment.

There is a need to provide a medical device that can be sterilized quickly and reliably, without any risk that the sterilization compromises the function of the medical device.

Herein is disclosed a sterilizable medical device. The device includes electronic circuitry and a radiation shield, including a coating. The coating is configured to protect the electronic circuitry from radiation for sterilization of the medical device, such as from a sterilizing dosage of charged particle beam radiation. The device can be sterilized by radiation with reduced risk of damage to the electronics. An interface can be coupled to a patient, e.g. by being attached to the patient's skin or invasively by being inserted into the patient's body. The coupling may involve tissue, blood, or a fluid for injection into the patient (e.g. an intraveneous fluid) or taken from the patient. The interface may be a surface of the medical device, e.g. an exterior surface or an interior surface of the medical device. Sterile coupling of the interface, e.g. exterior surface, to the patient reduces the risk of infection. The interface can be sterilizable, e.g. with charged particle beam radiation, such as electron beam radiation, e.g. for sterile coupling to a patient. The interface may, in another embodiment, be an interior surface, e.g. the inner wall of tubing through which a fluid is let to or from the patient.

Electronic devices may be adversely impacted by radiation, including radiation used for medical sterilization. Radiation may cause structural and/or functional changes in semiconductor materials, electronic circuitry, and/or transistors such as bipolar transistors. Radiation used for medical sterilization can include charged particle beams such as electron beams, which, along with other forms of radiation such as electromagnetic radiation (e.g. gamma rays), can damage and/or cause functional changes of electronic circuitry.

Medical devices described herein are radiation tolerant, including medical devices that include electronic circuits that can normally be damaged by radiation. For example, by providing a coating on the medical device that attenuates and/or scatters radiation, electronic circuits of medical devices can be protected from radiation induced damage, such as from medically sterilizing radiation. It is desirable to reduce the risk of malfunction, after radiation sterilization, of electronic medical devices. It is also desirable that the medical device provides a readable output or indication that the device has been sterilized, and/or that the device's electronics are not damaged or at least functioning within tolerances, e.g. by the sterilizing dosage of radiation. Alternatively/additionally, the medical device can provide a readable output or indication related to a sensing function of the device, e.g. an output representative of a pressure and/or temperature.

The device described above may further be improved by any of the features described herein, in any combination. Each of the following features is independent of the other features and can be combined with other features, such as to provide a functionality and/or technical advantage; such as protecting electronic circuitry from radiation, decreasing the size of the device, improving reliability, allowing for sterilization, or providing protection against counterfeit devices. Each of the features described herein may be used for improving the device, independent of whether a feature has been described within the context of a device or in the context of a function, process, or method. A device described herein can be configured to perform the processes and/or functions described herein. The method(s) described herein can be performed, in whole or in part, using the device(s), their features and/or functionalities.

Herein "sensor" may be used interchangeably with "sensor circuitry." Herein, the IC can be an application specific integrated circuit (ASIC). Herein, an IC and/or ASIC can include a field programmable gate array (FPGA), processor, and/or memory. Herein, the authenticator can be an n bit, n being an integer in particular of a power of 2, such as a 16, 32 or 64 bit authenticator. "Authenticator" and "authenticator circuit" may be used interchangeably herein. Herein, "device" may be used interchangeably with "medical device." Herein, "bridge" may be used interchangeably with "Wheatstone bridge," and "bridge circuit," which may be used as a sensor, such as a pressure sensor. Herein, a bridge may include at least one piezoresistor.

Herein, the IC and/or authenticator can be formed from one or more semiconductor chips.

Herein, the coating can attenuate radiation to protect bipolar junctions such as PN junctions of the electronic circuitry from the radiation. The radiation described herein, e.g. medically sterilizing radiation, may cause damage to electronic circuitry if incident on a semiconductor thereof, such as PN junction thereof, used in the electronic circuitry.

The medical device may include reference circuitry which is configured to e.g. indicate a completed sterilization. In one embodiment, the reference circuitry may be configured to have a state which is representative a dosage of the charged particle beam that has been received by the reference circuitry during sterilization of the medical device. For example, the base emitter voltage can provide a first output a first output before exposure to radiation and a second output after exposure, such as after exposure to a threshold dosage which may be a medically sterilizing dosage. The threshold dosage can be below a second threshold of damage to the electronic circuitry, which can also be detected by the reference circuitry. The reference circuitry can be configured to output a base emitter voltage, e.g. as an indicator of received radiation. The reference circuitry can be or include a radiation fuse sensor. The reference circuitry may be configured to be irreversibly changed by exposure to radiation, e.g. such that the output irreversibly changes with exposure.

The authenticator circuit described herein can be a digital key security chip.

Herein, medically sterilizing radiation can include any one or more of: charged particle beam radiation, electron beam radiation, proton beam radiation, or electromagnetic radiation such as gamma rays. Herein, attenuation of radiation may be by scattering, such as angular scattering of the radiation.

Herein, "high Z layer" means high atomic number layer; and "low Z layer" means low atomic number layer.

Herein, "MEMS" can be used for microelectromechanical system or microelectromechanical device, which are used interchangeably.

Herein, "and/or" means at least one of the listed elements. For example, "A and/or B" means: only A; only B, at least A; at least B; or at least A and B. For example, "X, Y, and/or Z" means: only X; only Y; only Z; at least X; at least Y; at least Z; only X and Y; only X and Z; only Y and Z; only X, Y, and Z; at least X and Y; at least X and Z; at least Y and Z; or at least X, Y, and Z. Herein, an "(s)" at the end of a word means one or more; for example a layer(s) is one or more layers.

Herein, reference numerals referred to in the description, which refer to features of the drawings, are an aid to understanding the invention and are not necessarily limiting.

Herein, "electrical circuitry" may exclude electronic circuitry that is sensitive to radiation. "Electrical circuitry" may be insensitive to radiation in comparison to "electronic circuitry." For example, "electrical circuitry" can exclude circuitry with one or more of: PN junctions, semiconductor amplifiers, or semiconductor transistors e.g. those used for signal processing. Electrical circuitry may include passive elements, such as resistors, capacitors, wires, and inductors. Electronic circuitry may include active elements, such as PN junctions, transistors, diodes, or integrated circuits that include semiconductors. Herein, electronic circuitry can include or be sensor circuitry, e.g. for sensing a pressure and/or temperature.

The sensor circuitry described herein may include thin or thick film resistors on a ceramic substrate. Herein, the sensor output can be a differential output, e.g. for reducing common mode noise.

The medical devices described herein can be critical medical devices or semi-critical medical devices.

Herein, a "critical medical device" can be a medical devices for the use of blood, blood products or other sterile medicinal products and/or sterile medical devices, and medical devices that are intended to penetrate the skin or mucous membranes and can come into contact with blood, internal tissues or organs, including wounds; for example, a blood pressure sensor (e.g. for arterial blood pressure sensing), a catheter, and an insulin pump. Herein, a "semi-critical medical device" can be a medical device that comes in contact with mucous membranes or skin, for example an endoscope. The medical devices described herein that can be sterilized by charged particle beams may be single use and/or disposable devices. Limiting a device to a single use can decrease infection risk. The medical device described herein may be a blood pressure sensor. The medical device described herein may be disposable, portable, wearable and/or invasive. The medical devices described herein are sterilizable. Herein, a "sterilizable medical device" may be sterilized, e.g. a medical device that has been subjected to a sterilizing dosage of radiation is sterilized and sterilizable. Herein, "medical device" and "sterilizable medical device" are used interchangeably. The medical devices described herein are sterilizable. Herein, coupling of an interface of a medical device to a patient can be via a direct or indirect coupling, e.g. through an intravenous tube, e.g. a tube (possibly connected to an artery for a medical device that measures arterial blood pressure), or a needle for subcutaneous delivery of a medicine.

Herein, the electronic circuitry, and/or housing thereof, can include a semiconductor package, semiconductor die, and/or circuit board.

The coating of the medical device can be configured to shield and/or protect the electronic circuitry from an electron beam, such as a medically sterilizing dosage, such as from an electron beam of up to 1 MeV, or up to 500 keV, or up to 300 keV, or up to 250 keV, or up to 200 keV. Electron beam radiation is a relatively common form of radiative medical sterilization for devices. It is desired to have available an electronic medical device that can be sterilized using electron beam radiation. The coating can be configured for scattering an electron beam. Materials for scattering an electron beam may be less expensive and easier to work with than materials that attenuate electron beams by other mechanisms. The coating can shield from other forms of sterilizing radiation, such as other charged particle beams, for example, protons.

The coating can be over the electronic circuitry, such as over a housing(s) of the electronic circuitry. The coating can surround the electronic circuitry, e.g. the housing(s) of the electronic circuitry. Alternatively/additionally, a first coating can cover the electronic circuitry from one side, and a second coating can cover the electronic circuitry from an opposite side, e.g. such that the electronic circuitry is surrounded by one or more coatings. The coating(s) can at least partially cover a substrate which may support the electronic circuitry.

The coating can be a multilayer coating. The effectiveness of scattering and/or attenuation of radiation for medical sterilization can be improved by using multiple layers, e.g. different layers that are each tuned to efficiently attenuating different kinds of radiation.

For example, the coating includes at least one high atomic number layer and at least one low atomic number layer. Low and high atomic number layers (low and high Z layers) can be stacked alternatingly. The high Z layer can be more effective at attenuating electrons and/or at least some types of electromagnetic radiation used for medical sterilization such as gamma rays. The low atomic number layer(s) can be effective at stopping protons. It can be desirable for the electronics of the device to be protected from more than one kind of radiation. Alternatively/additionally, the attenuation of radiation, e.g. from electrons, may be improved by multiple interfaces, in the coating, between different material types.

The high atomic number layer(s) can include a post-lanthanide element such as at least one of tungsten, tantalum or bismuth (e.g. as oxides), and the low atomic number layer(s) can include only pre-lanthanide elements, such as elements of the fifth period or lower, or of the fourth period or lower, or of the third period or lower. Post-lanthanide elements have relatively high atomic numbers and can be effective at blocking radiation. Tungsten, tantalum and bismuth are relatively inexpensive elements having a high atomic number, so the cost effectiveness of the material can be improved. Alternatively/additionally, tungsten, tantalum and bismuth can be relatively easily processed, for example as oxides. Alternatively/additionally, forming interfaces between high and low Z layers can improve scattering and/or attenuation of radiation, e.g. electron beam radiation. Bismuth, tantalum and tungsten have high atomic numbers and are capable of efficient attenuation of radiation, such as electron beam radiation. Bi, Ta and W in the form of oxides can improve biocompatibility.

The high Z layer(s) can include powders of particles, e.g. of at least one of bismuth oxide, tantalum oxide or tungsten oxide. Powders and/or particles can be easily handled. Alternatively/additionally, the particles of oxides can be dispersed into matrices and/or can be biocompatible.

The high Z layer(s) can include a matrix, such as at least one of a sol gel or epoxy. A sol gel or epoxy can form a film and can include the bismuth, tantalum and/or tungsten oxide power/particles, e.g. as inclusions. A matrix can improve biocompatibility and/or aid in dispersing powder and/or particulate forms of the high Z elements.

The low Z layer(s) can be electrically conductive, e.g. by containing or consisting of a conductive material such as a metal and/or a metal alloy. For example, the low Z layer(s) include aluminum, and/or is mainly aluminum. Aluminum is inexpensive and a low atomic number element. Aluminum can aid in stopping protons. The conductivity of aluminum may also aid in providing a sink for attenuated electrons.

Each layer of the multilayer coating can have a thickness of, for example, 50 µm to 4000 µm. For example, the low Z layer can be 50-500 µm, or 100-400 µm. For example, an aluminum layer of 100-400 µm can be used. The high Z layer can be 400-4000 µm. For example, a high Z layer that includes bismuth oxide in a matrix can be used, having a thickness of 400-2000 µm. The total thickness of the multilayer can be 2-50 mm. The high atomic number layer(s) can be formed from a soft gel and/or epoxy. Epoxies and gels can be easily used as coatings. Alternatively/additionally, oxide forms of high Z elements like tungsten, tantalum and bismuth can be readily incorporated into epoxies and gels, such as oxide based gels, such as those based on sol-gels.

The medical device can include a reference circuitry configured for providing a first output before exposure to radiation and a second output after exposure, such as after exposure to a threshold dosage, such as the sterilizing dosage. The reference circuitry can act as a sensor and/or fuse to allow the determination of exposure to radiation, such as whether a sterilizing dosage was received, or whether a dosage above a threshold associated with an increased risk of damage to the electronics was received. The reference circuitry can aid in determining the sterilization state and/or reliability of the device.

The medical device can include electrical and/or sensor circuitry, such as a pressure sensor and/or a temperature sensor, e.g. for blood pressure and/or temperature. Pressure and/or temperature measurements are highly useful in medicine.

The electrical circuitry can couple to the interface, e.g. electrically and/or mechanically. Such coupling can allow the electrical circuitry to perform functions at the interface, e.g. a sensing function or a mechanical function (e.g. a pump).

The electronic circuitry can include at least one of: an application specific integrated circuit (ASIC); or an authenticator. An ASIC can allow for flexible control of the device, such as programmability. An authenticator can provide a way to check for counterfeit devices. The ASIC can be coupled, communicatively and/or electrically, to the electrical and/or sensor circuitry. The ASIC can store data from the electrical and/or sensor circuitry. The ASIC can allow control, such as programmable control, of the electrical and/or sensor circuitry, e.g. by trimming resistors of sensor circuitry. The ASIC can include memory, such as flash memory, and/or a processor.

Electrical circuitry can be electromechanically coupled to an interface of the device which can be used for sterile coupling to a patient.

The ASIC can be stacked vertically with the authenticator. The ASIC can be stacked vertically with the electrical and/or sensor circuitry. Stacking can reduce the footprint of the device.

The electrical circuitry of the sterilizable medical device can include sensor circuitry, such as a pressure sensor and/or temperature sensor.

Sensor circuitry of the device can include a resistor bridge, such as a piezoresistive bridge, such as a Wheatstone bridge. A resistor bridge can provide a differential output. A resistor bridge can reduce unwanted common mode noise. Piezoresistors can be precisely controlled for resistance and/or be effective strain gauges, e.g. for determining pressure. Wheatstone bridges can provide differential output, reduce common mode noise, provide reliable measurement, be relatively radiation insensitive (e.g. compared to an electronic circuit that includes a PN junction), and/or be relatively easy to tune or implement.

The ASIC can be configured for controlling the electrical circuitry (e.g. the sensor circuitry), e.g. the resistor bridge, such as by trimming any one or more of the resistors. It can increase convenience and flexibility to have the ASIC configured to control the sensor circuitry and/or bridge.

The ASIC can be coupled to a microelectromechanical device that includes the electrical and/or sensor circuitry. A MEMS can allow for miniaturization of the device.

In the following, the invention is described in more detail by means of embodiments with reference to the attached figures. In the figures, features which correspond to one another in terms of structure and/or function can be provided with the same reference signs for convenience.

The combinations of features shown and described in embodiments are for explanatory purposes only. Herein, a feature of an embodiment can be omitted if its technical effect is not important for a particular application. A further feature can be added to an embodiment, e.g. if its technical effect should be advantageous or necessary for a particular application.

In the following, several examples are described.

In the figures:
- Fig. 1: shows a schematic of a medical device, according to embodiments;
- Fig. 2: shows a schematic of a medical device, according to embodiments;
- Fig. 3: shows a schematic of a medical device, according to embodiments;
- Fig. 4: shows a schematic of a medical device, according to embodiments;
- Fig. 5: shows a schematic of a medical device, according to embodiments;
- Fig. 6: shows a method of determining a state of a medical device, according to embodiments;
- Fig. 7: shows a schematic of a medical device, according to embodiments; and

The examples described herein are for illustrative purposes.

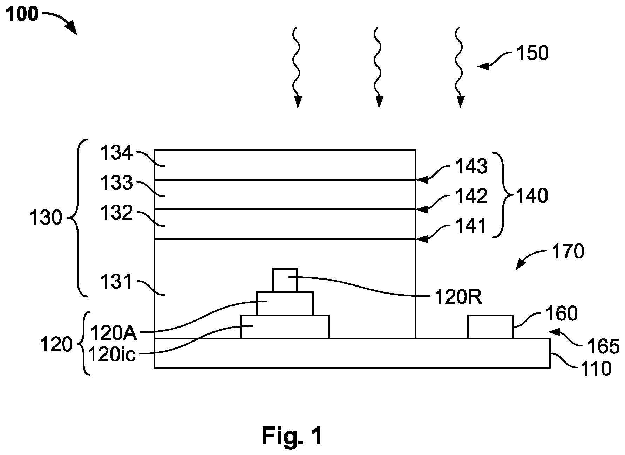

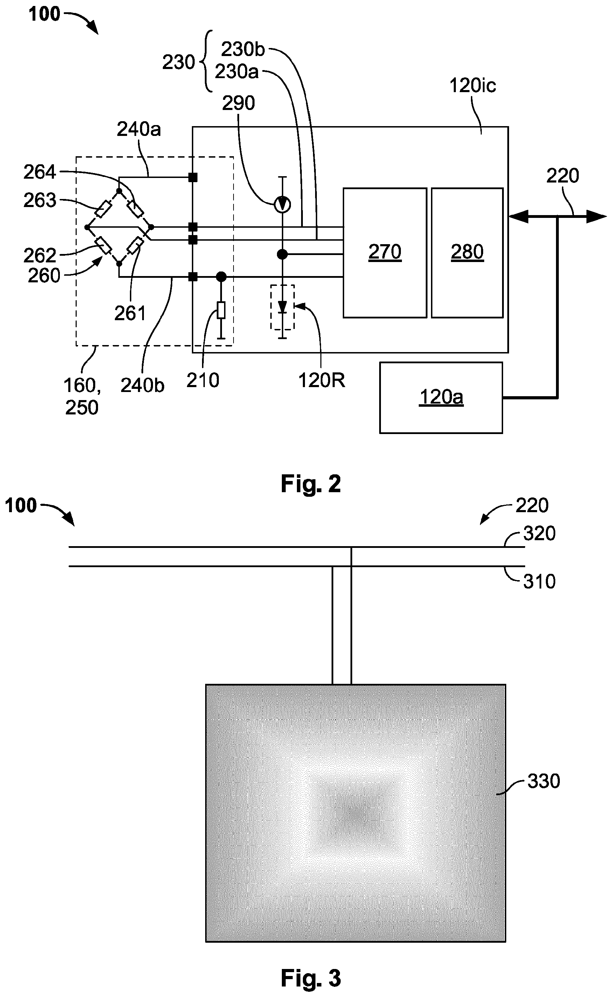

Fig. 1 shows a medical device 100, according to an embodiment. A medical device 100 can include electronic circuitry 120, such as at least one of an integrated circuit (IC) 120ic, e.g. an application specific integrated circuit; or an authenticator 120a. The IC 120ic and authenticator 120a can be stacked vertically, e.g. over a substrate 110 and/or electrical circuitry 160. Stacking can result in a smaller device, which can allow the device to reach into smaller body lumina, for example. A substrate 110 can provide at least partial rigidity to the device 100, and may also support electrical circuitry 160 and/or sensor circuitry 250 (see Fig. 2), especially a microelectromechanical device, e.g. for pressure and/or temperature sensing. Vertical stacking may reduce the size, e.g. the footprint of the device 100, and can allow the medical device to be incorporated into smaller devices and/or make the device 100 fit in smaller body lumina, e.g. for insertible and/or invasive medical devices. The electrical circuitry 160 may be part of a microelectromechanical system, MEMS, 165; e.g. a MEMS for sensing, such as determining pressure and/or temperature, such as determining the temperature or pressure of a fluid, such as a liquid, such as blood. Alternatively/additionally, a MEMS 165 can include a pump, e.g. a drug delivery pump (such as an insulin pump). For example, an interface 170 of the device 100, for sterile coupling to a patient, can be a surface of the MEMS 165. Alternatively/additionally, the electrical circuitry 160 can include an environmentally sensitive component at the interface 170 for sterile coupling to the patient, e.g. for providing a mechanical pumping force and/or sensing surface (e.g. a strain gauge and/or pressure sensor).

The electrical circuitry 160 and/or interface 170 for sterile contact with a patient may be uncoated or have a thinner coating than the electronic circuitry 120.

The electrical circuitry 160 may be formed as a unit, e.g. on a ceramic, glass, or silicon substrate, which may serve as the substrate 110 for the entire medical device 100. The electrical circuitry 160 can be stacked with the IC 120i and authenticator 120a, e.g. to save space. The electrical circuitry 160 may also be electrically and/or communicatively coupled to the IC 120ic.

The medical device can include a reference circuitry 120R, which may be partially protected from radiation 150, e.g. less protected from radiation 150 in comparison to the electronic circuitry 120, e.g. the IC 120ic and/or authenticator 120a. The reference circuitry 120R may be configured to provide a first output before exposure to radiation and a second output after exposure, such as after exposure to a threshold dosage of radiation 150, e.g. a sterilizing dosage. The reference circuitry 120R can include a bipolar transistor which can be sensitive to radiation 150. The bipolar transistor may provide the first and second outputs, which depend on exposure to radiation 150. For example, a base emitter voltage can provide the variable output which can be used as an indicator that the device 100 has been sterilized by radiation 150. The reference circuitry 120R output can be communicatively coupled to the IC 120ic.

Fig. 1 shows radiation 150, which can be used to medically sterilize the device 100, which may be tolerant to one or more forms of radiation used for medical sterilization. For example, the medical device 100 may be tolerant to charged particle beams such as proton beams and/or electron beams, and/or electromagnetic radiation (e.g. gamma rays). The medical devices 100 described herein can be tolerant to medically sterilizing dosages of radiation. For example, medically sterilizing electron beams can have energies of up 1 MeV, or up to 500 keV, or up to 300 keV, or up to 250 keV, or up to 200 keV; and the radiation protective coating 130 described herein can protect the electronic circuitry 120 from the radiation 150, e.g. the electron beam.

The electrical circuitry 160 may be less shielded from radiation 150 than the electronic circuitry 120, e.g. the IC 120ic and/or authenticator 120a. The electrical circuitry 160 may be more environmentally sensitive, e.g. for sensing the environment and/or electrically and/or mechanically coupling to the environment.

The electronic circuitry 120 may include more radiation sensitive electronic components, such as bipolar transistors and/or other semiconductor components. At least part of the electrical circuitry 160 may be located at an interface 170, e.g. an interface 170 for sterile coupling to a patient. Electrical circuitry 160 at the interface 170, surface, or near the interface can provide for better coupling to the patient and/or environment. Better coupling can result in a more responsive and/or accurate sensor, for example. The electrical circuitry 160 can be configured to measure pressure such as blood pressure. Having the electronic circuitry 120 more shielded, in comparison to the interface 170 and/or electrical circuitry 160, can allow the more radiation sensitive electronic components to be protected from radiation 150, while allowing better coupling (e.g. electrical and/or mechanical) between the electrical circuit 160 and the patient, e.g. via the interface 170, which can be sterile or sterilizable. The electrical circuitry 160 may be less radiation sensitive than the electronic circuitry 120.

The electronic circuitry 120 may be a mixed signal circuit, e.g. one with analog circuits and digital circuits. For example, the electronic circuitry 120 may be coupled to the electrical circuitry 160 for receiving and/or transmitting analog signals. The electronic circuitry 120 may be communicatively coupleable via an at least partial digital interface, e.g. IIC (inter-integrated circuit), such as for data output.

The coating 130 can attenuate radiation, such as electrons of an electron beam 150, e.g. by scattering the beam, e.g. angularly scattering the beam. As shown in Fig. 1, the coating 130 can include a multilayer coating; e.g. a plurality of layers 131, 132, 133, 134 stacked on top of each other. The layers 131, 132, 133, 134 are referred to herein as first through fourth layers. The materials and functions of the layers 131, 132, 133, 134 can vary as described herein. The layers 131, 132, 133, 134 can form a multilayer sandwich structure.

There can be any number of layers 131, 132, 133, 134 to make up the coating 130. In an embodiment that can be combined with any other embodiment described herein, the coating 130 includes alternating layers. Adjacent layers of the coating 130 can have different properties, structure, and/or composition. Such differences can enhance the attenuation of radiation, such as by allowing different layers to be tuned for effecting attenuation of different types of radiation. Alternatively/additionally, the interfaces 140, where adjacent layers meet, as illustrated in Fig. 1 may enhance attenuation of radiation 150, such as electron beam radiation and/or protons or other charged particles, e.g. by inducing scattering.

For example, the coating 130 can include a plurality of high atomic number layers (e.g. the second and fourth 132, 134) and a plurality of low atomic number layers (e.g. the first and third 131, 133). The low and high atomic number layers can be stacked alternatingly.

The average atomic number of the elements making up the high atomic number layer(s) can be higher than the average atomic number of the elements making up the low atomic number layer(s). For example, the high atomic number layer(s) include a post-lanthanide element such as tungsten, tantalum or bismuth, and the low atomic number layer(s) include only pre-lanthanide elements, such as elements of the fifth period or lower, or of the fourth period or lower, or of the third period or lower. A high atomic number layer, compared to a low atomic number layer, can be more effective at attenuation of electrons and/or electromagnetic radiation such as gamma rays. A low atomic number layer can be effective at attenuation of protons. Alternatively/additionally, the interfaces 140 can be effective at attenuating radiation 150, such as electron beam radiation and/or proton beams.

The high atomic number layer(s) can include an oxide, glass, gel, or epoxy. The high atomic number layer(s) can include bismuth oxide, tantalum oxide and/or tungsten oxide. High atomic number elements such as bismuth, tantalum and tungsten can efficiently attenuate radiation. Oxides may be relatively easy to handle, such as in powder or particulate form, which can be included in a matrix such as an epoxy or sol gel.

The high atomic number layer(s) can include bismuth and oxygen, such as bismuth oxide, tantalum oxide and/or tungsten oxide. The low atomic layer(s) can include a conductive material, such as a metal, for example aluminum.

Bismuth, tantalum and/or tungsten are relatively inexpensive and have a high atomic number. Bismuth, tantalum and tungsten can be included in an oxide, glass, and/or gel. Gels and glasses can simplify the manufacturing and/or reduce difficulties with possible delamination. Oxides can be inexpensive and, herein, need not be crystalline. The coating 130 can be made from alternating layers that include, respectively, aluminum and bismuth oxide, materials which may be available at low cost.

Alternatively/additionally, the layers can include at least one proton attenuation layer and at least one electron attenuation layer. The coating 130 can include alternating layers of proton attenuation and electron attenuation layers. For example, the second and fourth layers 132, 134 are electron attenuation layers and the first and third 131, 133 layers are proton attenuation layers. A proton attenuation layer can be more efficient at attenuation protons than an electron layer; and an electron attenuation layer can be more efficient at attenuation electrons. A coating with both a proton attenuation layer(s) and an electron attenuation layer(s) can efficiently attenuate both protons and electrons. A coating 130 that attenuates both electrons and protons efficiently may be more tolerant to a wider range of possible radiation sterilization sources.

For example, as shown in Fig. 1, the coating 130 can extend laterally (perpendicular to the thickness direction of the coating) beyond the edges of the electronic circuitry 120, or housing(s) thereof.

The coating 130 can surround the electronic circuitry 120, e.g. a housing(s) of the electronic circuitry 120. Alternatively/additionally, a first coating can cover the electronic circuitry 120 from one side and a second coating can cover the electronic circuitry from an opposite side, e.g. such that the electronic circuitry is surrounded by one or more coatings. The coating(s) 130 can at least partially cover the optional substrate 110 which may support the electronic circuitry 120. A second coating can be on the opposite side of the substrate 110, e.g. for surrounding the electronic circuitry 120. The edges of the optional second coating can extend laterally (in a plane perpendicular to the thickness direction of the multilayer) beyond the edges of the electronic circuitry 120, e.g. beyond the edges of the housing(s) of the electronic circuitry 120.

Fig. 2 shows a medical device 100, according to an embodiment, such as according to the embodiments explained with reference to Fig. 1. Fig. 2 does not show the coating 130. The electric 160 and/or sensor circuitry 250 of the medical device 100 can include a bridge circuit 260, such as a resistor bridge, e.g. a piezoresistive bridge, such as a Wheatstone bridge. The bridge circuit 260 may have four resistors 261, 262, 263, 264, such as they are shown. An output of the bridge circuit 260 can be configured to provide the sensor output 230, e.g. through electrical connections 231, 232. The sensor output 230, e.g. differential output, can be configured for communicative coupling, e.g. to the IC 120ic, as shown, through electrical leads 230a and 230b; and may optionally be processed by the IC 120ic before being output from the device 100. The bridge circuit 260 can be electrically connected to leads for power, e.g. 240a for ground, and 240b for positive or negative bias. A power source 290, such as a battery or coupler configured to receive external power for the device 100, may be coupled directly or indirectly to the bridge 260 of the sensor circuitry 250, e.g. through an analog front end 270, which may be included in the IC 120ic. The power source 290 can be separate from the IC 120ic, or be part of the IC 120ic.

The sensor output 230, e.g. of the bridge circuit 260, can be for determining a pressure, such as a pressure as determinable by a variable resistance of at least one of the resistors 261. At least one of the resistors 261 can be a piezoresistor which is sensitive to pressure and/or deformation, e.g. configured to provide a pressure sensitive resistance. Herein, a sensing resistor(s) is at least one resistor with variable resistance according to an environmental factor such as pressure and/or temperature. The resistors 261, 262, 263, 264 can be trimmed, for example by the electronic circuitry 120, e.g. the IC 120ic, which may be electrically coupled to the resistors 261, 262, 263, 264 for trimming their respective resistances. The ability to trim the resistors 261, 262, 263, 264 can enhance the sensitivity of the sensor circuitry 250 and/or correct instrumental drift. Trimming by the IC 120ic can allow better control compared to physically trimming the resistors 262, 263, 264, e.g. by laser removal of material of the resistors which is irreversible. Coupling the electronic circuitry 120, e.g. the IC 120ic, to a micromechanical device (MEMS) that includes the sensor circuitry 250, can allow control of the sensor circuitry 250.

Alternatively/additionally, the IC 120ic can compensate for relatively wide manufacturing tolerances of the sensor circuitry 250, such as by controlling at least one tunable parameter of the sensor circuitry 250, e.g. a resistance. For example, sensor circuitry 250 that includes a MEMS, can be tuned by an electrically connected IC 120ic, e.g. ASIC, which can compensate for relatively wide manufacturing tolerances of the MEMS, including resistors thereof, e.g. piezoresistors.

The sensor output 230 of the sensing circuitry 160 and/or bridge 260 can be input into the electronic circuitry 120, e.g. input into the IC 120ic. For example, the IC 120ic can amplify the sensor output 230 and optionally further process the sensor output 230. The device 100 can include a communication coupling 220, such as to provide communication between the electronic circuitry 120 and an external device such as a computer. For example, the communication coupling 220 includes a serial communication bus. For example, an I2C bus can be used for the communication coupling 220. An I2C bus provides flexibility for programming the electronic circuitry 120, e.g. the IC 120ic, which may include a logically programmable device, e.g. a FPGA and/or ASIC. The communication coupling 220 can alternatively/additionally be coupled to an authenticator circuit 120a. An authenticator circuit 120a can allow users to identify counterfeit devices which may not pass rigorous safety standards for medical devices (or at least allow users to confirm authentic devices).

The medical device 100 can include a temperature sensor 210, such as a temperature sensitive resistor, which may be located on the electrical circuitry 160 or on the IC 120ic. A temperature sensor 210 on or near the same MEMEs as the electrical circuitry 160 can provide more responsive temperature measurements of the electrical circuitry 160.

The temperature sensor 210 can be communicatively and/or electrically coupled to the IC 120ic and/or electrical circuitry 160. For example, a radiation insensitive temperature sensor 210 can be on the electrical circuitry 160, e.g. a thermistor. A thermistor can be less radiation sensitive than a bandgap temperature sensor. The semiconductor materials of a bandgap temperature sensor can be altered by radiation, changing the properties of the sensor. The temperature sensor 210 may be communicatively coupled, directly or indirectly, such as via the communication coupling 220.

The device 100 can include, e.g. in the IC 120ic, a digital block 280 which may be logically programmable. The digital block 280 may allow modification of feedback algorithms for temperature compensation, trimming of one or more resistors 261, 262, 263, 264, and/or adjustment of settings for a reference circuitry 120R. The IC 120ic, e.g. the digital block 280 thereof, can control the power line voltage 240b to the sensor circuitry 160, bridge circuit 260, temperature sensor 210, and/or reference circuitry 120R. Alternatively/additionally, the IC120ic, e.g. the digital block 280, may include logic which processes the data, signals, and/or outputs, e.g. from the electrical circuitry 160, reference circuitry 120R, and/or temperature sensor 210. Raw or processed data can be output via the communication coupling 220; e.g. by applying a temperature compensation which depends on the output of the temperature sensor 210.

In an example, the reference circuit 120R is communicatively coupled indirectly to the communication coupling 220. The IC 120ic, such as with the digital block 280, can be electrically coupled to the output of the reference circuit 120R. The IC 120ic and/or digital block 280 can provide a data output via the communication coupling 220 which depends on the output of the reference circuit 120R. For example, the IC 120ic and/or digital block 280 provides a data output, such as a Boolean, to indicate whether the device 100 has been sterilized with radiation. Alternatively/additionally, the reference circuit 120R can act as a fuse to indicate exposure to a threshold amount of radiation, e.g. a sterilizing dosage or a threshold for damaging the electronic circuitry 120. The reference circuit 120R can be configured for providing a first output before exposure to sterilizing radiation; a second output after exposure, such as after exposure to a threshold dosage which is suitable for sterilization of the device 100. The second output may also be at a dosage below a second threshold for reaching an unacceptable risk of damage to the electronic circuitry 120. The reference circuit 120R can provide a third output when the dosage of radiation 150 is above a second threshold at which the device 100 has received a dosage which results in damage to the electronic circuitry 120 or is such that there is an unacceptably high risk of radiation induced damage to the electronic circuitry 120.

The IC 120ic, e.g. via the digital block 280 and/or analog front end 270 can be configured to control the sensor circuitry 250, such as by controlling variable resistor(s) of the bridge circuit 260. The sensor circuitry 250 can include variable resistors electrically coupled to the electronic circuitry 120, e.g. the IC 120ic, which may be an ASIC. Alternatively/additionally, the power lines 240a, 240b for the electrical circuitry 160 and/or the output 230 of the sensor circuitry 250 can be electrically coupled to the electronic circuitry 120, e.g. the IC 120ic.

Fig. 3 illustrates, schematically, according to an embodiment, a medical device 100, such as the medical device as described with respect to Figs. 1 and/or 2. The communication coupling 220 can be an IIC bus, which may include a serial data line 310 and a serial clock line 320. The footprint 330 of the device can include space for the electronic circuitry 120 and electrical circuitry 160. The size of the device 100 and/or the footprint 330 of the device 100 can be reduced by vertically stacking at least two of: the electrical circuitry 160, the authenticator 120a, or the IC 120ic. For example, at least the authenticator 120a and IC 120ic are stacked vertically.

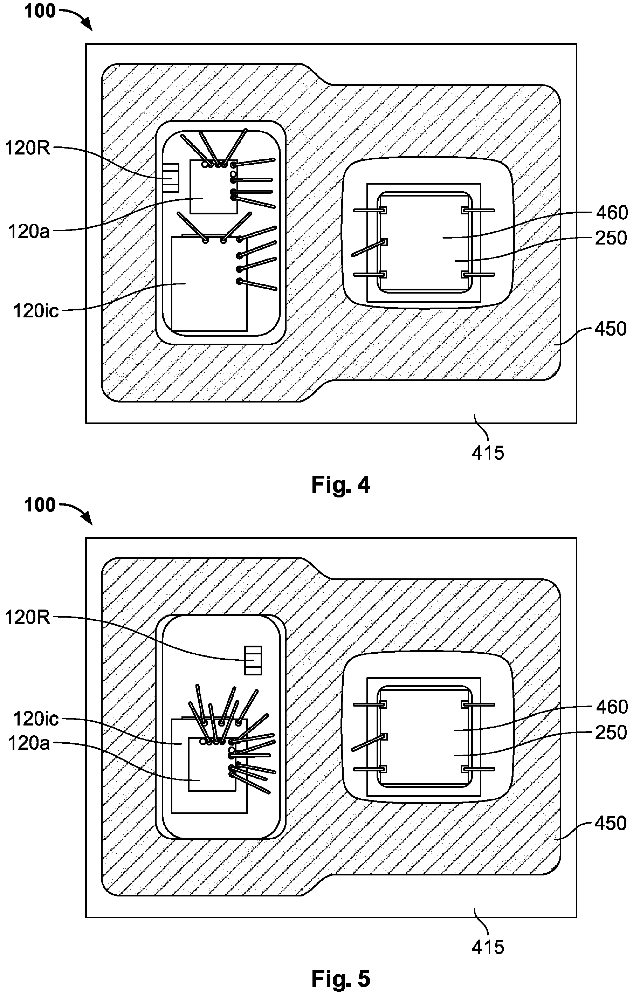

Fig. 4 illustrates a medical device 100 according to an embodiment, such as the medical device 100 as described with respect to Figs. 1, 2, and/or 3. The device 100 can include sensor circuitry 250, a reference circuit120R, an IC 120ic, and an authenticator 120a. The device 100 can include a housing 450 that can aid in protecting the components from the environment. The sensor circuitry 250 can include or be supported by a substrate 460, which can be ceramic. For example, the sensor circuitry 250 includes film resistors (e.g. piezoresistors) on the substrate 460. The sensor substrate 460 can be fixed to a device substrate 415. The device substrate 415 can also support the housing 450, which can be attached, e.g. bonded, such as with epoxy. The electronic circuitry 120 and/or reference circuit 120R can be supported directly or indirectly on the device substrate 415. For example, the authenticator circuit 120a and IC 120ic are each supported by the device substrate 415. Having the authenticator circuit 120a and IC 120ic each supported by the device substrate 415 can allow for the electronics of each to be located farther under the coating 130 (not shown in Fig. 4), such as to improve the attenuation of radiation 150.

Fig. 5 illustrates a medical device 100 according to an embodiment, such as according to the medical device as described with respect to Figs. 1, 2, 3, and/or 4. The device 100 of Fig. 4 differs from that of Fig. 4 in the placement of the authenticator circuit 120a, which is stacked on the IC 120ic. The size, e.g. the footprint 330 of the device can be reduced by stacking components.

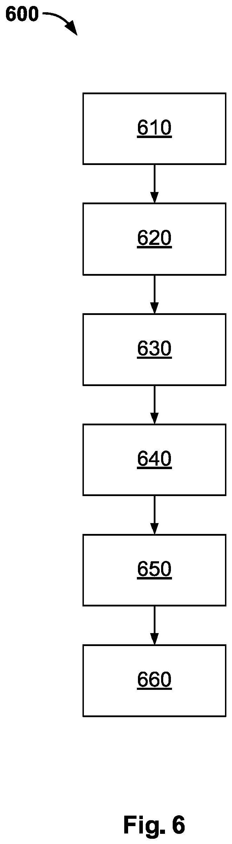

Fig. 6 illustrates a method of determining a state of a medical device, according to an embodiment.

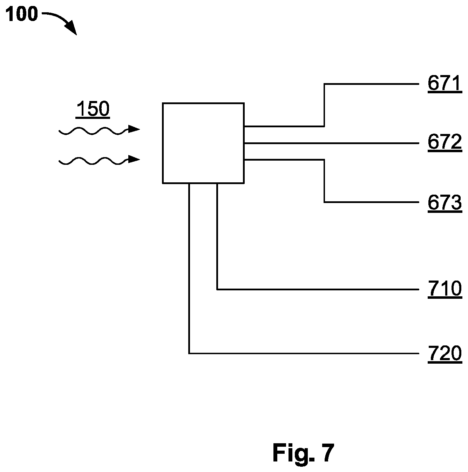

Fig. 7 illustrates the medical device, according to an embodiment.

The embodiments described with respect to Figs. 6 and 7 are embodiments in accordance with the other embodiments described herein, e.g. with respect to the other figures. The method 600 can include any one or more of: communicatively coupling 610 the device 100 for reading an output, e.g. an output of a reference circuit 120R; reading 620 a first output 671 (e.g. and determining the state to be unsterilized); exposing 630 the device 100 to medically sterilizing radiation, e.g. a above a threshold and/or dosage for sterilization and below a second threshold and/or dosage for damage of the electronics; reading 640 a second output 672 (e.g. and determining the state to be sterilized); exposing 650 the device 100 to radiation above a threshold for damage; reading 660 a third output 673 (e.g. and determining the device to have a risk of being electronically damaged).

Alternatively/additionally, the device 100 may generate a readable output without communicative coupling 610, e.g. with a display or LED. Communicative coupling 610 may be done out of sequence, such as after any exposure step. The device 100 and the reference circuit 102R may be calibrated so that the output of the reference circuit at the first reading 620 is unnecessary.

The use of the device may be such that the first radiation exposure is already enough to cause damage, so that reading 640 the second output 672, is possibly skipped. The steps described as reading steps may be replaced by steps for generating the outputs, and/or storing data based on the outputs. For example, the data generated as an output can be stored in the integrated circuit 120ic, which may be an ASIC. The data generated as an output can be first processed before being stored. The output(s) that is read after communicative coupling 610 can be from the stored data of the IC 120ic.

For example, if one exposure provides a dosage sufficient to expose the reference circuit 120R to the second threshold, then the third output 673 is read 660 after only one exposure, which corresponds to irradiation sufficient to damage the electronics and put the reference circuit 120R into a state indicative of damage. In another example, the radiation dosage is too low to sterilize the device 100, and the output generated or stored from the reference circuitry 120R is not the second output 672 indicative of sterilization. In such a case, the output can be the first output 671, indicative of an unsterilized state. The outputs 671, 672, 673 should not necessarily be construed to occur strictly in sequence for the device 100, since the output depends on the extent of radiation exposure.

The medical device 100 may have a second kind of output, e.g. an output related to the determination by the sensor circuitry 160, e.g. of pressure and/or temperature. The IC 120ic may read and/or store data from the sensor circuitry 160. The data from the sensor circuitry 160 can be communicatively coupled and read. In an embodiment, the sensor circuitry 160 provides an output for determining pressure, such as blood pressure.

The medical device 100 described herein can be a blood pressure sensor for medical use, and can be designed for invasive use, e.g. by being inserted into the patient's body and making contact with tissue, blood, or a sterile medicine to be delivered to a patient (e.g. intraveneously or subcutaneously). The device 100 may be configured to come into contact with fluid and/or tissue that is directed into or out of the body of the patient. The device 100 can be disposable. The device 100 can provide a sensor output 710 (e.g. a blood pressure), and optionally an authentication output 720, e.g. from an authenticator circuit 120a. The device 100 can provide, e.g. as determined by a reference circuitry 120Rs, an indicator and/or output related to detection of a radiation dosage, e.g. at least two of: a first, second, and third output 671, 672, 673, each respectively related to a detected radiation dosage. The medical device 100 described herein can be sterilizable in its entirety, e.g. the entire surface of the device can be sterilized using charged particle beam radiation, such as an electron beam. The interface 170 of the device 100 can be an exterior surface of the device 100 (such as for providing an environmentally sensitive interface for a sensor circuit 250 of the device 100), or can be an interior surface (e.g. making contact with a reservoir of sterile medicine for delivery to a patient).

**REFERENCE NUMERALS**

| | |
|---|---|
| medical device | 100 |
| substrate | 110 |
| electronic circuitry | 120 |
| authenticator | 120a |
| integrated circuit (IC) | 120ic |
| reference circuitry | 120R |
| radiation protective coating | 130 |
| first layer | 131 |
| second layer | 132 |
| third layer | 133 |
| fourth layer | 134 |
| interfaces | 140 |
| first interface | 141 |
| second interface | 142 |
| third interface | 143 |
| radiation | 150 |
| electrical circuitry | 160 |
| microelectromechanical system | 165 |
| interface | 170 |
| temperature sensor | 210 |
| communication coupling | 220 |
| sensor output | 230 |
| output+ | 230a |
| output- | 230b |
| power - and/or ground line | 240a |
| power line + | 240b |
| sensor circuitry | 250 |
| bridge circuit | 260 |
| resistor 1 | 261 |
| resistor 2 | 262 |
| resistor 3 | 263 |
| resistor 4 | 264 |
| analog front end | 270 |
| digital block | 280 |
| power source | 290 |
| serial data line | 310 |
| serial clock line | 320 |
| footprint | 330 |
| device substrate | 415 |
| device housing | 450 |
| sensor substrate | 460 |
| method of state determination | 600 |
| communicatively coupling | 610 |
| reading a first output | 620 |
| exposing to sterilizing radiation | 630 |
| reading a second output | 640 |
| exposing to damaging radiation | 650 |
| reading a third output | 660 |
| first output | 671 |
| second output | 672 |
| fhird output | 673 |
| sensor output | 710 |
| authentication output | 720 |

## Claims

1. A sterilizable medical device (100), comprising
electronic circuitry (120);
a radiation shield (130), including a coating (130),
the coating being configured to protect the electronic circuitry (120) from a dosage of charged particle beam radiation (150) for sterilizing the medical device; and
an interface (170) configured for sterile coupling to a patient.

2. The sterilizable medical device (100) of claim 1, wherein
the medical device (100) is a medical device selected from the group containing: a semi critical medical device, a critical medical device, an invasive medical device, a one-way medical device and a one-way invasive medical device.

3. The sterilizable medical device (100) of any claim 1 or 2, wherein
the coating (130) is a multilayer coating (131, 132, 133, 134).

4. The sterilizable medical device (100) of claim 3, wherein
the multilayer coating (131, 132, 133, 134) includes a plurality of high atomic number layers (132, 134) and a plurality of low atomic number layers (131, 133), the low and high atomic number layers being stacked alternatingly; wherein
the low atomic number layer is optionally electrically conductive, comprising an electrically conductive metal.

5. The sterilizable medical device (100) of claim 4, wherein
the high atomic number layers (132, 134) include a post-lanthanide element such as tungsten, tantalum or bismuth, and
the low atomic number layers (131, 133) include only pre-lanthanide elements, such as elements of the fifth period or lower, or of the fourth period or lower, or of the third period or lower.

6. The sterilizable medical device (100) of claim 5, wherein
the high atomic number layers (132, 134) include at least one of bismuth oxide, tantalum oxide and tungsten oxide; and
the low atomic number layers (131, 133) include aluminum.

7. The sterilizable medical device (100) of claim 6, wherein
the high atomic number layers (132, 134) include powders or particles of at least one of bismuth oxide, tantalum oxide or tungsten oxide.

8. The sterilizable medical device (100) of any one of claims 5-7, wherein
the high atomic number layers (132, 134) include a matrix such as a sol gel and/or epoxy.

9. The sterilizable medical device (100) of any one of claims 1-8, further comprising:
reference circuitry (120R) configured for providing a first output (671) before exposure to the charged particle beam radiation (150) and a second output (672) after exposure, such as after exposure to a threshold dosage, such as the sterilizing dosage.

10. The sterilizable medical device (100) of any one of claims 1-9, further comprising:
electrical circuitry (160) which couples to the interface (170), the coupling being electrical and/or mechanical.

11. The sterilizable medical device (100) of any one of claims 1-10, wherein
the electronic circuitry (120) includes at least one of:
an application specific integrated circuit, ASIC, (120ic); or an authenticator (120a); wherein
the ASIC is coupled, communicatively and/or electrically, to the electrical circuitry (160).

12. The sterilizable medical device (100) of claim 11, wherein
the ASIC (120ic) is stacked vertically with the authenticator (120a); wherein optionally,
the ASIC (120ic) is stacked vertically with the electrical circuitry (160).

13. The sterilizable medical device (100) of claim 11 or 12, wherein
the electrical circuitry (160) includes sensor circuitry (250), such as a pressure sensor and/or a temperature sensor (210); wherein optionally
the sensor circuitry (250) includes
a resistor bridge, such as a piezoresistive bridge, such as a wheatstone bridge.

14. The sterilizable medical device (100) of claim 13, wherein
the ASIC (120ic) is configured for controlling the electrical circuitry (160), such as the resistor bridge, such as by trimming any one or more of the resistors (261, 262, 263, 264).

15. The sterilizable medical device (100) of any one of claims 13-14, wherein
the ASIC (120ic) is coupled to a microelectromechanical device (165) that includes the sensor circuitry (250).

## Patentansprüche

1. Sterilisierbares medizinisches Gerät (100), umfassend:
eine elektronische Schaltung (120);
eine Strahlungsabschirmung (130), die eine Beschichtung (130) aufweist,
wobei die Beschichtung so ausgeführt ist, dass sie die elektronische Schaltung (120) vor einer Dosis einer Bestrahlung mit einem geladenen Teilchenstrahl (150) zum Sterilisieren des medizinischen Gerätes schützt; sowie
eine Schnittstelle (170), die zum sterilen Koppeln mit einem Patienten ausgeführt ist.

2. Sterilisierbares medizinisches Gerät (100) nach Anspruch 1, wobei das medizinische Gerät (100) ein medizinisches Gerät ist, das aus der Gruppe ausgewählt wird, die ein semikritisches medizinisches Gerät, ein kritisches medizinisches Gerät, ein invasives medizinisches Gerät, ein medizinisches Einweggerät und ein invasives medizinisches Einweggerät einschließt.

3. Sterilisierbares medizinisches Gerät (100) nach einem der Ansprüche 1 oder 2, wobei die Beschichtung (130) eine Mehrfachbeschichtung (131, 132, 133, 134) ist.

4. Sterilisierbares medizinisches Gerät (100) nach Anspruch 3, wobei
die Mehrfachbeschichtung (131, 132, 133, 134) eine Vielzahl von Schichten mit hoher Ordnungszahl (132, 134) sowie eine Vielzahl von Schichten mit niedriger Ordnungszahl (131, 133) umfasst, wobei die Schichten mit niedriger und hoher Ordnungszahl abwechselnd übereinandergeschichtet sind; und wobei
die Schicht mit niedriger Ordnungszahl optional elektrisch leitfähig ist und ein elektrisch leitfähiges Metall umfasst.

5. Sterilisierbares medizinisches Gerät (100) nach Anspruch 4, wobei
die Schichten mit hoher Ordnungszahl (132, 134) ein Post-Lanthanoid-Element, beispielsweise Wolfram, Tantal oder Wismut, einschließen, und
die Schichten mit niedriger Ordnungszahl (131, 133) nur Prä-Lanthanoid-Elemente, beispielsweise Elemente der fünften Periode oder niedriger, oder der vierten Periode oder niedriger, oder der dritten Periode oder niedriger, einschließen.

6. Sterilisierbares medizinisches Gerät (100) nach Anspruch 5, wobei
die Schichten (132, 134) mit hoher Ordnungszahl wenigstens eines von Wismutoxid, Tantaloxid oder Wolframoxid einschließen; und
die Schichten mit niedriger Ordnungszahl (131, 133) Aluminium einschließen.

7. Sterilisierbares medizinisches Gerät (100) nach Anspruch 6, wobei die Schichten (132, 134) mit hoher Ordnungszahl Pulver oder Teilchen wenigstens eines von Wismutoxid, Tantaloxid oder Wolframoxid einschließen.

8. Sterilisierbares medizinisches Gerät (100) nach einem der Ansprüche 5 - 7, wobei die Schichten (132, 134) eine Matrix, beispielsweise ein Sol-Gel und/oder Epoxid, einschließen.

9. Sterilisierbares medizinisches Gerät (100) nach einem der Ansprüche 1 - 8, das des Weiteren umfasst:
eine Referenzschaltung (120R), die so konfiguriert ist, dass sie einen ersten Ausgang (671) vor Bestrahlung (150) mit dem geladenen Teilchenstrahl und einen zweiten Ausgang (672) nach Bestrahlung, beispielsweise nach Bestrahlung mit einer Schwellendosis, beispielsweise der Sterilisierungsdosis, bereitstellt.

10. Sterilisierbares medizinisches Gerät (100) nach einem der Ansprüche 1 - 9, das des Weiteren umfasst:
eine elektrische Schaltung (160), die mit der Schnittstelle (170) gekoppelt ist, wobei die Kopplung elektrisch und/oder mechanisch ist.

11. Sterilisierbares medizinisches Gerät (100) nach einem der Ansprüche 1 - 10, wobei
die elektronische Schaltung (120) wenigstens aufweist:
eine anwendungsspezifische integrierte Schaltung, ASIC, (120ic); oder einen Authentifikator (120a); wobei
die ASIC kommunizierend und/oder elektrisch mit der elektrischen Schaltung (160) gekoppelt ist.

12. Sterilisierbares medizinisches Gerät (100) nach Anspruch 11, wobei die ASIC (120ic) vertikal mit dem Authentifikator (120a) übereinandergeschichtet ist; wobei optional die ASIC (120ic) vertikal mit der elektrischen Schaltung (160) übereinandergeschichtet ist.

13. Sterilisierbares medizinisches Gerät (100) nach Anspruch 11 oder 12, wobei
die elektrische Schaltung (160) eine Sensorschaltung (250) aufweist, beispielsweise einen Drucksensor und/oder einen Temperatursensor (210); wobei optional
die Sensorschaltung (250) aufweist:
eine Widerstandsbrücke, beispielsweise eine piezoresistive Brücke, beispielsweise eine Wheatstone-Brücke.

14. Sterilisierbares medizinisches Gerät (100) nach Anspruch 13, wobei die ASIC (120ic) zum Steuern der elektrischen Schaltung (160), beispielsweise der Widerstandsbrücke, ausgeführt ist, beispielsweise durch Abgleichen eines oder mehrerer der Widerstände (261, 262, 263, 264).

15. Sterilisierbares medizinisches Gerät (100) nach einem der Ansprüche 13 - 14, wobei die ASIC (120ic) mit einem mikroelektromechanischen Bauelement (165) gekoppelt ist, das die Sensorschaltung (250) aufweist.

## Revendications

1. Dispositif médical stérilisable (100), comprenant
des circuits électroniques (120) ;
un écran anti-rayonnement (130), incluant un revêtement (130),
le revêtement étant configuré pour protéger les circuits électroniques (120) contre une dose de rayonnement de faisceau de particules chargées (150) destinée à stériliser le dispositif médical ; et
une interface (170) configurée pour un couplage stérile à un patient.

2. Dispositif médical stérilisable (100) selon la revendication 1, dans lequel le dispositif médical (100) est un dispositif médical sélectionné dans le groupe comprenant : un dispositif médical semi-critique, un dispositif médical critique, un dispositif médical invasif, un dispositif médical unidirectionnel et un dispositif médical invasif unidirectionnel.

3. Dispositif médical stérilisable (100) selon l'une quelconque des revendications 1 ou 2, dans lequel
le revêtement (130) est un revêtement multicouche (131, 132, 133, 134).

4. Dispositif médical stérilisable (100) selon la revendication 3, dans lequel
le revêtement multicouche (131, 132, 133, 134) inclut une pluralité de couches à nombre atomique élevé (132, 134) et une pluralité de couches à nombre atomique bas (131, 133), les couches à nombre atomique bas et élevé étant empilées de manière alternée ; dans lequel
la couche à nombre atomique bas est facultativement électriquement conductrice, comprenant un métal électriquement conducteur.

5. Dispositif médical stérilisable (100) selon la revendication 4, dans lequel
les couches à nombre atomique élevé (132, 134) incluent un élément post-lanthanide tel que le tungstène, le tantale ou le bismuth, et
les couches à nombre atomique bas (131, 133) incluent uniquement des éléments prélanthanides, tels que des éléments de la cinquième période ou moins, ou de la quatrième période ou moins, ou de la troisième période ou moins.

6. Dispositif médical stérilisable (100) selon la revendication 5, dans lequel
les couches à nombre atomique élevé (132, 134) incluent au moins un parmi l'oxyde de bismuth, l'oxyde de tantale et l'oxyde de tungstène ; et
les couches à nombre atomique bas (131, 133) incluent de l'aluminium.

7. Dispositif médical stérilisable (100) selon la revendication 6, dans lequel les couches à nombre atomique élevé (132, 134) incluent des poudres ou des particules d'au moins un parmi l'oxyde de bismuth, l'oxyde de tantale ou l'oxyde de tungstène.

8. Dispositif médical stérilisable (100) selon l'une quelconque des revendications 5 à 7, dans lequel
les couches à nombre atomique élevé (132, 134) incluent une matrice telle qu'une matrice sol-gel et/ou époxy.

9. Dispositif médical stérilisable (100) selon l'une quelconque des revendications 1 à 8, comprenant en outre :
des circuits de référence (120R) configurés pour fournir une première sortie (671) avant l'exposition au rayonnement de faisceau de particules chargées (150) et une seconde sortie (672) après l'exposition, comme après l'exposition à une dose de seuil, comme la dose de stérilisation.

10. Dispositif médical stérilisable (100) selon l'une quelconque des revendications 1 à 9, comprenant en outre :
des circuits électriques (160) qui se couple à l'interface (170), le couplage étant électrique et/ou mécanique.

11. Dispositif médical stérilisable (100) selon l'une quelconque des revendications 1 à 10, dans lequel
les circuits électroniques (120) incluent au moins un parmi :
un circuit intégré spécifique à une application, ASIC, (120ic) ; ou un authentificateur (120a) ; dans lequel
l'ASIC est couplé, en communication et/ou électriquement, aux circuits électriques (160).

12. Dispositif médical stérilisable (100) selon la revendication 11, dans lequel l'ASIC (120ic) est empilé verticalement avec l'authentificateur (120a) ; dans lequel facultativement,
l'ASIC (120ic) est empilé verticalement avec les circuits électriques (160).

13. Dispositif médical stérilisable (100) selon la revendication 11 ou 12, dans lequel
les circuits électriques (160) incluent des circuits de capteur (250), tel qu'un capteur de pression et/ou un capteur de température (210) ; dans lequel facultativement
les circuits du capteur (250) incluent
un pont de résistance, tel qu'un pont piézorésistif, tel qu'un pont de wheatstone.

14. Dispositif médical stérilisable (100) selon la revendication 13, dans lequel l'ASIC (120ic) est configuré pour commander les circuits électriques (160), tels que le pont de résistance, par exemple en ajustant une ou plusieurs des résistances (261, 262, 263, 264).

15. Dispositif médical stérilisable (100) selon l'une quelconque des revendications 13 à 14, dans lequel
l'ASIC (120ic) est couplé à un dispositif micro-électromécanique (165) qui inclut les circuits de capteur (250).
